# EUROPEAN PATENT APPLICATION

(11) **EP 4 332 241 A1**
(43) Date of publication of application: **06.03.2024**
(21) Application number: 22382818.7
(22) Date of filing: 01.09.2022
(51) Int. Cl.: C12Q 1/6886

(54) **SINGLE NUCLEOTIDE POLYMORPHISM FOR THE PROGNOSIS OF BREAST CANCER**

(71) Applicant: Consejo Superior de Investigaciones Cientificas, 28006 Madrid (ES)
(72) Inventor: Alarcón Sánchez, Balbino José, 28049 Madrid (ES); Hortal Borowski, Alejandro Miguel, 28049 Madrid (ES); Cifuentes Caballero, Claudia, 28049 Madrid (ES)
(74) Representative: Pons

(57) **Abstract**

The present invention discloses prognosis methods in breast cancer, particularly the methods of the invention use the SNP at rs8570 for the prognosis of a patient suffering from breast cancer or for assessing the risk of developing breast cancer in a subject. The SNP rs8570 is located the 3'-UTR of the RRAS2 gene and can be determined and genotyped in an isolated biological sample of a patient for prognosis of breast cancer or for assessing the risk of developing breast cancer in a subject. The invention also provides a kit for detecting and genotyping the SNP rs8570.

## Description

The present invention generally relates to the field of biomedicine and, more specifically, to prognosis methods in breast cancer, particularly to the use of a single nucleotide polymorphism (SNP) in the mRNA of the *RRAS2* gene or the DNA of the RRAS2 gene for the prognosis of a patient suffering from breast cancer or for assessing the risk of developing breast cancer in a subject.

### BACKGROUND ART

Among the different kinds of cancer, breast cancer is the most common malignant tumor in women and the leading cause of cancer death in women since breast cancer accounts for about 30% of female cancers, and has a mortality-to-incidence ratio of 15%. Numerous genes are mutated in breast cancer being the most representative the mutations affecting *BRCA1, BRCA2, PALB2, CDH1* among others. Moreover, the disease is clinically divided into three main subtypes by hormone receptor (ER and PR) and HER2 (ERBB2) status: luminal ER-positive and PR-positive, which is further subdivided into luminal A and B; HER2-positive; and triple-negative breast cancer (TNBCS). Due to these characteristics, breast cancer is a heterogeneous disease in terms of genotypes, clinical manifestations, prognosis and sensitivity or resistance to existing medical treatments (Loibl, S, et al. The Lancet, 2021 (10286), 1750-1769).

In recent years, numerous publications have shown that new high-performance techniques in genomics can be very useful for the diagnosis, the prognosis or for predicting the risk of suffering from breast cancer. Specifically, the study of SNPs that reside in different locations of the genome and their association with diagnosis, prognosis and prediction of risk of cancer has been extensively studied in the context of breast cancer.

The rs743554 SNP, which is located in the 3'-UTR region of the ITGB4 gen, was found to be associated with a poor prognosis in breast cancer (Brendle A et al. Carcinogenesis. 2008; 29: 1394-1399). EP2446056A2 disclosed a BRCAI polymorphic signature that indicates an increased risk for developing breast or ovarian cancer, wherein the signature comprises the determination of the presence or absence of the single nucleotide polymorphisms (SNPs) rs8176318 and rsl060915, wherein the presence of these SNPs indicates an increased risk for developing breast or ovarian cancer. The SNP rs10878441 located in the LRRK2 gene was found to be associated with poor prognosis of breast cancer in a Chinese population. Stratified analyses demonstrated that rs10878441 was related to breast cancer prognosis in grade II patients and lymph node-negative patients. (Zhang L, et al. Aging. 2021;13(5):7465-7480).

However, due to the complexity of breast cancer there is still a need in the field to provide new single nucleotide polymorphisms , specially in those genes that have a pro-active effect in the development of breast cancer, and that allow to establish the prognosis of breast cancer in a simple and reliable way.

### DESCRIPTION OF THE INVENTION

The inventors have identified that a single nucleotide polymorphism (SNP) rs8570 located in the 3'-UTR of the gene *RRAS2* is associated with a higher risk of developing breast cancer and a poorer prognosis if it is found in the tumour samples, independently of the surrounding tissue genotype.

The single nucleotide polymorphism (SNP) rs8570 of the gene RRAS2 was first described as one of the 105 SNPs of genes in the MAPK pathway associated with cutaneous melanoma. However, no significant association was found between the SNP and the melanoma susceptibility or its progression (Odds Ratio= 0.95) (Liu H, et al. Carcinogenesis. 2013 Apr; 34(4): 885-892). However, in a recent publication (Hortal AM, et al. Mol Cancer 2022; 21, 35) this SNP was associated associated with higher expression of RRAS2 in leukemic cells of CLL patients. However, the high frequency of CLL leukemias that did not bear the SNP and had exclusively the G allele (51%), compared to the expected frequency of 46% for a random distribution, gave this SNP little predictive value in CLL.

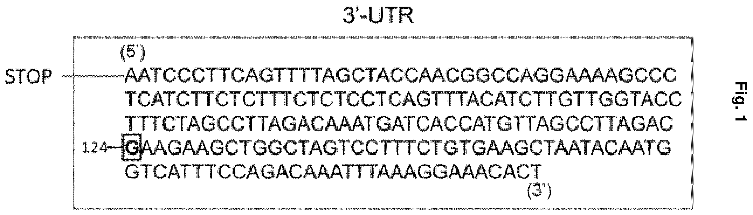

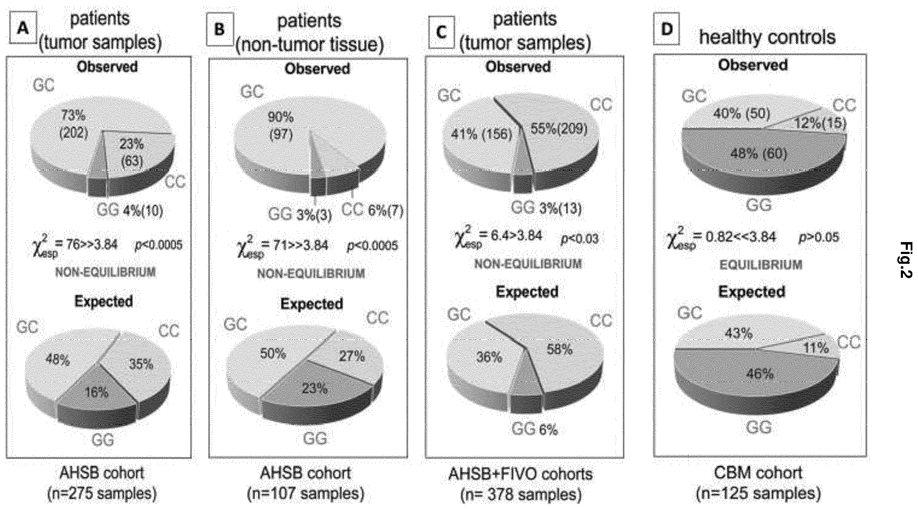

As the examples of the present invention show, the inventors sequenced by Sanger method the 3'-UTR of the DNA of the gene *RRAS2* in breast cancer samples of a cohort of 378 patients finding that in some patients there was a single nucleotide polymorphism in the SNP rs8570, characterized by having a C nucleotide in position 124 after the stop codon in the 3'UTR of the gene *RRAS2* (whereas the canonical sequence contains a G in that position, see fig.1). Particularly, they found that 209 out of a total of 378 sequenced samples (a 55%) had C at the SNP position in both chromosomal gene alleles, whereas 156 (a 41%) were heterozygotes (GC) at the SNP position and only 13 (a 3%) was homozygote for the canonical allele (fig.2C). Furthermore, the inventors also found that the expression of the C allele in homozygosity (CC) at the SNP rs8570 position is associated with higher proliferation rate and therefore more aggressive disease in breast cancer. Altogether, these results associate expression of the C allele in homozygosis in the SNP rs8570 of the mRNA of the gene *RRAS2* with higher *RRAS2* expression in the tumors and with poorer prognosis.

Based on these findings, the inventors have found that the SNP rs8570 can be used for the prognosis of a patient suffering from breast cancer or for assessing the risk of developing breast cancer in a subject.

Thus, a first aspect of the present invention relates to an *in vitro* use of the single nucleotide polymorphism (SNP) rs8570 of the mRNA of the gene *RRAS2* or of the genomic DNA of the gene *RRAS2* for the prognosis of a patient suffering from breast cancer, or for assessing the risk of developing breast cancer in a subject.

As it is used in the present invention, DNA of the gene *RRAS2* refers to the genomic DNA (gDNA) of the gene *RRAS2* or complementary DNA of the gene *RRAS2* (cDNA). cDNA is synthesized from a single-stranded RNA (e.g., messenger RNA (mRNA) or microRNA (miRNA)) template in a reaction catalyzed by the enzyme reverse transcriptase.

As it is used in the present description, the term "single nucleotide polymorphism" or "SNP" means a single nucleotide (A, C, T or G) position in a genomic sequence for which two or more alternative alleles are present at an appreciable frequency (e.g., at least 1%) in a human population. In the present invention, the terms polymorphic site and SNPs can be used indistinctly. The SNPs are typically named using the accession number in the Single Nucleotide Polymorphism (SNP) database (dbSNP) at National Center for Biotechnology Information (NCBI) accessible at http://www.ncbi.nlm.nih.gov/projects/SNP/. Alternatively, the SNP can be named as well by indicating the position at the gene or genomic contig wherein the variation occurs, and the type of nucleotide change that occurs at said position or the type of amino acid change in the polypeptide encoding by said gene. The SNP rs8570 can be found in the Single Nucleotide Polymorphism (SNP) database (dbSNP) at National Center for Biotechnology Information (NCBI) as "rs8570".

The single nucleotide polymorphism rs8570 is located in human chr11 : 14279213 (GRCh38.p13) and it is comprised within the 3'-UTR region of the gDNA of the *RRAS2* gene and its mRNA. This gene encodes a member of the R-Ras subfamily of Ras-like small GTPases which is involved in the regulation of MAPK signalling pathways. The SNP rs8570 genotype that is linked to a poor prognosis of breast cancer consists in a replacement of G of the canonical sequence in the 3'-UTR of *RRAS2,* at nucleotide 124 downstream of the stop codon, by C. The SNP rs8570 is located at the position 124 of the SEQ ID NO:1, where said sequence corresponds to the 3'-UTR region of the mRNA or gDNA of the *RRAS2* gene (fig. 1).

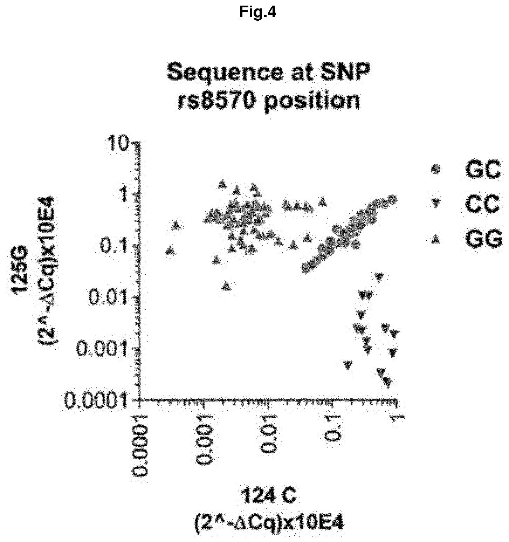

The genotype of the SNP rs8570 can be G in both chromosomal alleles (GG, homozygosis), C in both chromosomal alleles (CC, homozygosis) or G for one chromosomal allele and C for the other chromosomal allele (GC, heterozygosis) (fig.4). In the context of the present invention, the CC genotype is indicative of a poor breast cancer prognosis.

As used herein, and confined to diploids, "homozygote", "homozygosis" or "homozygous" is defined as a genetic condition existing when identical alleles reside at corresponding loci on homologous chromosomes.

As used herein, and confined to diploids, "heterozygote", "heterozygosis" or "heterozygous" is defined as a genetic condition existing when different alleles reside at corresponding loci on homologous chromosomes.

The term "prognosis" used in the present invention, refer to prediction of the future development or course of a disease, preferably a neoplastic disease, more preferably cancer, in particular breast cancer, including but without limitations, relapses or capability of metastatic dissemination.

The term "poor prognosis" or "unfavourable evolution" refers to not obtaining beneficial or desired clinical results which can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable leading therefore to a faster progression of the disease, lower overall survival defined as the percentage of patients who survive after diagnosis of cancer, relapse or recurrence.

The term "increased risk" refers to an increased or higher likelihood of developing breast cancer. Contrary to that, "decreased risk" refers to decreased or lower likelihood of developing breast cancer.

The term "subject" or "patient", as used in the present description, refers to a male or female human of any age and race.

In the context of the present invention the term "breast cancer" includes any type of neoplasm of breast. Preferably, the term "breast cancer" refers to a ductal adenocarcinoma of the molecular types: luminal A breast cancer, luminal B breast cancer, Her2+ breast cancer or triple negative breast cancer (TNBC).

The term "Her2" refers to receptor tyrosine-protein kinase erbB-2. The term "Her2+ breast cancer" refer to a breast cancer that is Her2 positive.

The term "luminal A breast cancer" includes breast cancers that are estrogen receptor-positive and progesterone receptor-positive, HER2-negative.

The term "Luminal B breast cancer" includes breast cancers that are estrogen receptor-positive and HER2-negative.

The term "Her2+ breast cancer" includes breast cancers that are estrogen receptor-negative and progesterone receptor-negative and Her2 positive.

The term "triple-negative breast cancer" includes breast cancers that are estrogen receptor-negative, progesterone receptor-negative, and HER2-negative.

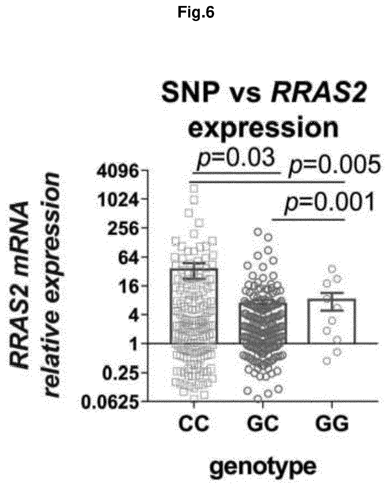

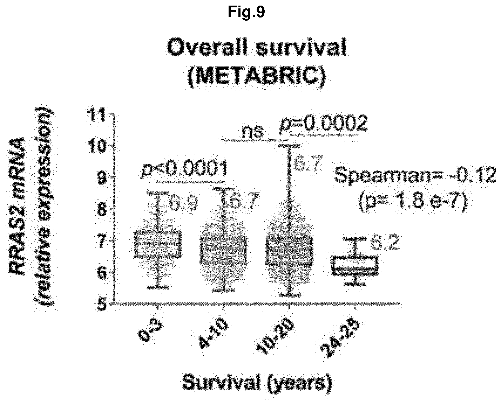

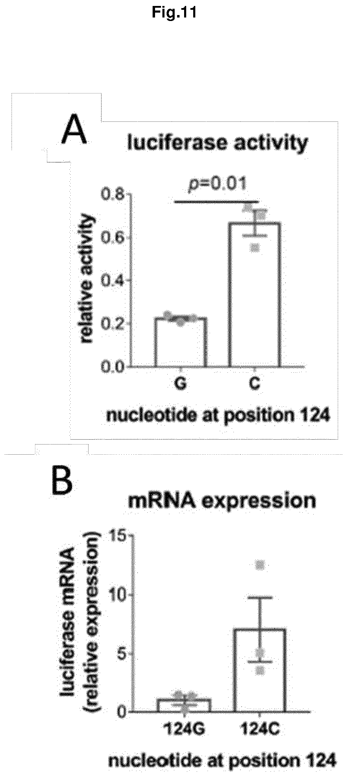

Overexpression of *RRAS2* is associated with a lower overall survival and poor prognosis (Fig.9) in breast cancer patients and the inventors have found that the overexpression of *RRAS2* is linked to the presence of the SNP rs8570, more specifically the overexpression of RRAS2 is linked to the presence of the allele C at the SNP rs8570 position (fig.11-12). The genotype CC at rs8570 showed the highest expression of *RRAS2* mRNA (fig.6).

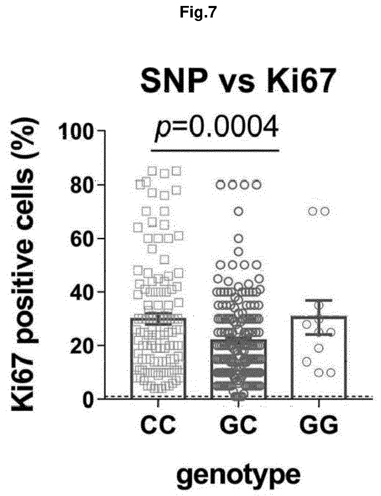

Genotype CC is also associated to a higher expression of Ki67 which is a commonly used cellular marker for cell proliferation (fig.7) Another marker of poor prognosis is the loss of the tumor suppressor p53, being the genotype CC more represented in tumors that are negative for p53, wherein said protein is mutated.

Therefore, the genotype CC at the SNP rs8570 is associated with higher *RRAS2* expression in the tumors, higher proliferation rate, more aggressive disease and poorer prognosis.

The inventors also found that, contrary to the genotype CC at rs8570, the genotype GG at the SNP rs8570 provides protection against developing breast cancer. Proof of that is the very low frequency (4%) of the GG genotype within the cohort of breast cancer patients.

A second aspect of the present invention relates to an *in vitro* method for the prognosis of a patient suffering from breast cancer, comprising the following steps:
i) detecting and genotyping the SNP rs8570 of the mRNA of the gene RRAS2 or of the gDNA of the gene *RRAS2* in an isolated biological sample of said patient suffering from breast cancer;
ii) classifying the subject into a group of poor prognosis when at least one allele at SNP rs8570 of the *RRAS2* gene is C.

In a preferred embodiment of the second aspect, the subject is classified into a group of poor prognosis in step ii) when the genotype at SNP rs8570 of the *RRAS2* gene is CG or CC.

In a preferred embodiment of the second aspect, the genotype CG at SNP rs8570 of the gene *RRAS2* indicates a poor prognosis with respect to the genotype GG at SNP rs8570 of the gene *RRAS2*; and the genotype CC at SNP rs8570 of the gene *RRAS2* indicates poorer prognosis with respect to the genotype CG.

Therefore, a poorer prognosis is established according to the genotypes at SNP rs8570 of the gene *RRAS2* following the next order GG<GC<CC, wherein CC indicates the poorest prognosis.

In a preferred embodiment of the second aspect, the subject is classified into a group of poor prognosis in step ii) when the genotype at SNP rs8570 of the *RRAS2* gene is CC.

In the methods of the present invention, the biological sample contains a nucleic acid, for example DNA, genomic DNA (gDNA), complementary DNA (cDNA), RNA or mRNA.

In a preferred embodiment of the second aspect, the biological sample contains gDNA, cDNA and/or mRNA from breast tumor cells.

The term "breast tumor cells" refers to the tumor cells that lead to the development of breast cancer.

The terms "biological sample" and "sample", used interchangeably in the present invention, relate to any sample which can be obtained from the subject.

In a preferred embodiment of the second aspect the biological sample is selected form the list consisting of biopsy sample, tissue, cell or fluid sample. In a preferred embodiment the biological sample is a tumoral sample, epidermal tissue, mucosal sample , whole blood, blood cells, serum, plasma, saliva, sputum, urine or breast exudate.

The detection of SNPs can be performed by means of multiple processes known by the person skilled in the art. Systems and methods for the detection of polymorphisms associated to genes include, but are not limited to hybridization methods and array technology, techniques based on mobility shift in amplified nucleic acid fragments, Loop-mediated isothermal amplification methods (LAMP), allele specific-LAMP amplification (AS-LAMP), reverse transcriptase-LAMP (RT-LAMP), oligonucleotide hybridization technique, Locked nucleic acid hybridization (LAN), Single Stranded Conformational Polymorphism (SSCP), denaturing gradient gel electrophoresis (DGGE), Chemical mismatch cleavage (CMC), Restriction fragment polymorphisms (RFLPs), nucleic acid sequencing method and the like. Generation of nucleic acids for analysis from samples generally requires nucleic acid amplification. Many amplification methods rely on an enzymatic chain reaction (such as a polymerase chain reaction). Real-time PCR (also known as Quantitative PCR, Real-time Quantitative PCR, or RTQ- PCR) is a method of simultaneous DNA quantification and amplification. DNA is specifically amplified by polymerase chain reaction. After each round of amplification, the DNA is quantified. Common methods of quantification include the use of fluorescent dyes that intercalate with double-strand DNA and modified DNA oligonucleotides (called probes or primers) that fluoresce after hybridising with a complementary DNA.

In a preferred embodiment of the methods of the invention, the detection and genotyping of the SNP is performed out by means of quantitative polymerase chain reaction (qPCR), Real-Time PCR (RT-qPCR), Retro-Transcriptase PCR (RT-PCR), long-range PCR, Restriction Fragment Length Polymorphism-PCR (PCR-RFLP), Loop-mediated isothermal amplification (LAMP), allele specific-LAMP amplification (AS-LAMP), reverse transcriptase-LAMP (RT-LAMP), DNA microarray, RNA microarray, oligonucleotide hybridization technique, Locked nucleic acid hybridization (LAN) and/or sequencing method, preferably wherein the sequencing method is Sanger method.

In a preferred embodiment of the methods of the invention, the nucleic acid sequencing method is the Sanger method.

In a preferred embodiment of the methods of the invention, the detection and genotyping of the SNP rs8570 is carried out by means of RT-qPCR. In a preferred embodiment, the methods of the present invention comprise detection and genotyping of the SNP rs8570 by means of RT-qPCR that comprises the use of the mismatching oligonucleotides that preferably comprise the sequences of table 1. In a preferred embodiment, the methods of the present invention comprise detection and genotyping of the SNP rs8570 by means of RT-qPCR that comprises the use of the mismatching oligonucleotides that consist of the sequences of table 1. These oligonucleotides can be designed as described in (Lefever S, et al. Cost-effective and robust genotyping using double-mismatch allele-specific quantitative PCR. Sci Rep. 2019;9:2150).

**Table 1. Preferred primers for genotyping the SNP rs8570 in RRSA2 by means of RT-qPCR.**

| Primer | Allele detection | ID | Sequence (5'->3') |
|---|---|---|---|
| Forward | G | SEQ ID NO: 2 | GAT CAC CAT GTT AGC CTT ATA CG |
| | C | SEQ ID NO: 3 | GAT CAC CAT GTT AGC CTT ATA CC |
| Reverse | G and C | SEQ ID NO: 4 | AGC AGC CTT AGT GTT TCC TT |

PCR reactions are preferably performed using 30 cycles of 45 seconds at 95ºC, 45 seconds at 60ºC and 2 minutes at 72ºC.

A third aspect of the invention relates to an *in vitro* method for assessing the risk of developing breast cancer in a subject, comprising the following steps:
i) detecting and genotyping the SNP rs8570 of the mRNA of the gene *RRAS2* or of the gDNA of the gene *RRAS2* in an isolated biological sample of said subject;
ii) classifying the subject into a group of increased risk of developing breast cancer when at least one alle at SNP rs8570 of the RRAS2gene is C.

In a preferred embodiment of the third aspect, the subject is classified into a group of increased risk of developing breast cancer in step ii) when the genotype at SNP rs8570 of the *RRAS2* gene is CG or CC.

In a preferred embodiment of the third aspect the step ii) further classifies the subject into a group of decreased risk of developing breast cancer when the genotype at SNP rs8570 of the *RRAS2* gene is GG.

In a preferred embodiment of the third aspect, the genotype CG at SNP rs8570 of the gene *RRAS2* indicates an increased risk of developing breast cancer with respect to the genotype GG at SNP rs8570 of the gene *RRAS2*; and the genotype CC at SNP rs8570 of the gene *RRAS2* indicates an increased risk of developing breast cancer with respect to the genotype CG.

Therefore a higher risk of developing breast cancer is established according to the genotypes at SNP rs8570 of the gene *RRAS2* GG<GC<CC, wherein CC indicates the highest risk.

In a preferred embodiment of the third aspect, the subject is classified into a group of increased risk of developing breast cancer in step ii) when the genotype at SNP rs8570 of the *RRAS2* is CC.

In a preferred embodiment of the third aspect, the subject has not been previously diagnosed with breast cancer.

In a preferred embodiment of the third aspect the biological sample is selected form the list consisting of biopsy sample, tissue, cell or fluid sample. In a preferred embodiment the biological sample is an epidermal tissue, mucosal sample, whole blood, blood cells, serum, plasma, saliva, sputum, urine or breast exudate.

The fourth aspect of the invention relates to a kit, hereinafter "the kit of the invention", for assessing the risk of developing breast cancer in a subject or for the prognosis of a patient suffering from breast cancer, wherein the kit comprises oligonucleotides and probes specific for the detection and genotyping of the SNP rs8570 of the mRNA of the gene *RRAS2* or the DNA of the gene *RRAS2,* preferably wherein the kit comprises the oligonucleotides comprising, more preferably consisting of, the SEQ ID NO: 2, SEQ ID NO: 3 and SEQ ID NO: 4.

In general, the kit of the invention comprises all necessary reagents for carrying out the previously described methods of the invention. The kit can further include, without any type of limitation, buffers, enzymes, as well as, for example, but without limitation, polymerases, cofactors for obtaining an optimal activity thereof, agents for preventing contamination, etc. On the other hand, the kit can include all of the necessary supports and containers for the start-up and optimization thereof. The kit can further contain other molecules, genes, proteins or probes of interest, which function as positive and negative controls.

The fifth aspect of the invention relates to the *in vitro* use of the kit of the invention for predicting the risk of developing breast cancer in a subject or for the prognosis of a patient suffering from breast cancer.

### DESCRIPTION OF THE DRAWINGS

**Fig. 1****.** Schematic representation of the rs8570 location in the 3'UTR of *RRAS2* sequence. SEQ ID NO: 1. Canonical sequence of 3'UTR of *RRAS2* with a G at position 124 after stop codon (the stop codon is TAG).
**Fig. 2****.** Observed versus the Expected distribution of GG, GC and CC genotypes at position of the SNP rs8570. A) Pie chart representation of the Observed versus the Expected distribution of GG, GC and CC genotypes at position of the SNP rs8570 in tumor samples AHSB cohort of breast cancer patients. The chi-squared value _{X}2 is greater than 3.84 and so the hypothesis that the Observed and Expected distributions are equivalent is rejected with a p<0.0005. B) Pie chart representation of the Observed versus the Expected distribution of GG, GC and CC genotypes at position of the SNP rs8570 in non-tumoral surrounding mammary tissue samples from AHSB cohort of breast cancer patients. The chi-squared value _{X}2 is greater than 3.84. C) Pie chart representation of the Observed versus the Expected distribution of GG, GC and CC genotypes at position of the SNP rs8570 in tumor samples from integrated AHSB plus FIVO cohorts of breast cancer patients. The chi-squared value _{X}2 is greater than 3.84 and so the hypothesis that the Observed and Expected distributions are equivalent is rejected with a p<0.03. D) Pie chart representation of the Observed versus the Expected distribution of GG, GC and CC genotypes at position of the SNP rs8570 in blood samples from CBM cohort of healthy volunteers. The chi-squared value _{X}2 is smaller than 3.84 and so the hypothesis that the Observed and Expected distributions are equivalent is not rejected.
**Fig. 3****.** SNP rs8570 tumor tissue VS non-tumor tissue. Enumeration of somatic mutations detected at the SNP position in tumor samples from AHSB cohort when compared with matched non-tumoral surrounding mammary tissue.
**Fig. 4****.** Allele distribution at SNP rs8570. Dot plot shows the differential Cq amplification by qPCR analysis of specific 124G and 124C primers compared to the expression of RRAS2 measured by its constitutive exons 3 and 4, AHSB cohort.
**Fig. 5****.** mRNA expression of RRAS2 in breast cancer patients. Scatter plot showing all points and median value of RT-qPCR data of RRAS2 mRNA expression in breast cancer patients from AHSB+FIVO cohorts classified according to their molecular classification. Percentage indicates the proportion of patients in each group overexpressing RRAS2 compared with non-tumoral tissue.
**Fig. 6****.** mRNA expression of RRAS2 vs genotype at rs8570. Scatter plot representing all points and median value of RT-qPCR RRAS2 mRNA expression in breast tumors from AHSB+FIVO cohorts according to GG, GC genotype at position of the SNP rs8570. Two-tailed unpaired t-test with Welch's correction.
**Fig. 7****.** Ki67 expression vs genotype at rs8570. Scatter bar plot showing all points and median value of the percentage of Ki67 positive breast cancer cells in AHSB+FIVO cohorts of patients harboring two G alleles or a G and a C allele. Two-tailed unpaired t-test with Welch's correction.
**Fig. 8****.** RRAS2 mRNA expression in breast cancer patients from AHSB cohort classified according to their p53 mutation status (negative or positive). Two-tailed unpaired t-test with Welch's correction.
**Fig. 9****.** Metadata analysis of RRAS2 relative expression and survival time. Data were obtained from the METABRIC database.
**Fig. 10****.** Schematic representation of the rs8570 location in the 3'UTR of RRAS2 sequence of the reporter plasmid. 124 nucleotides after the STOP codon (UAG). Above, canonical sequence with a G allele. Below, 3'-UTR with the non-canonical C allele in 124 position.
**Fig. 11.** A) Luciferase activity according to the allele at rs8570. Scatter bar plot representing luciferase relative activity in CAL-51 cells transfected with a luciferase reporter followed by RRAS2 3'UTR containing G or C allele. B) Luciferase mRNA according to the allele at rs8570. Quantification of luciferase mRNA relative expression in CAL-51 cells transfected with a luciferase reporter followed by RRAS2 3'UTR with G or C allele.

### EXAMPLES

These specific examples provided serve to illustrate the nature of the present invention and are only included for illustrative purposes, and therefore, they should not be interpreted as limiting the invention claimed herein. Thus, the examples described below illustrate the invention without limiting the field of application thereof.

### Example 1. Distribution of alleles at SNP rs8570 in the 3'-untranslated region (UTR) of RRAS2 gene.

Samples and data from patients included in this study were provided by Biobanks integrated in the Spanish National Biobanks Network, including the Biobank of the Aragon Health System, AHSB cohort 275 samples (PT17/0015/0039), and the Biobank FIVO , FIVO cohort 107 samples (PT17/0015/0051). Samples were processed following standard operating procedures with the appropriate approval of the Ethics and Scientific Committees. Samples were processed with the approval of the Ethical and Scientific Review Boards. 1ml of TRIzol^{®} (Invitrogen) was added per OCT-embedded sample and each sample was passed through a 18-gauge sterile needle. All samples were eluted in 30µl of nuclease-free water (Qiagen).

A cohort of 125 human blood samples (CBM cohort) was obtained from capillary blood of healthy volunteers after providing their informed consent for genomic DNA extraction. PBMCs were isolated from capillary blood of healthy volunteers by digital puncture and extraction of 20-30 µl of blood after signing an informed consent. Erythrocytes were lysed by treatment with ACK lysis buffer for 4 minutes. Genomic DNA was isolated following the phenol-chloroform protocol.

Genomic DNA obtained from capillary blood of healthy donors and OCT-samples from breast cancer samples were used to sequence by Sanger method the 3'UTR region of RRAS2, wherein said region encompasses the SNP rs8570 which rs8570 consists in a replacement of G of the canonical sequence in the 3'-UTR of RRAS2 at nucleotide 124 downstream of the stop codon by C (figure 1). A nested PCR was performed for this purpose using the oligonucleotides of table 2, using the oligonucleotide aligning to exon 3 of RRAS2 of SEQ ID NO:5, and the oligonucleotide aligning to RRAS2 3'-UTR as Rv of SEQ ID NO: 7 in the first PCR. For the nested PCR, the product of the first PCR was used as template and the utilized oligonucleotides were SEQ ID NO: 6 aligning to exon 5 of RRAS2, as Fw with the same Rv (SEQ ID NO: 7) oligonucleotide as in the first PCR. The product of this second PCR was sent to sequence by the Sanger method (Eurofins Genomics). Both PCR reactions were performed using 30 cycles of 45 s at 95 °C, 45 s at 60 °C and 2 min at 72 °C with PfuTurbo DNA Polymerase.

**Table 2. Oligonucleotides for the nested PCR**

| Primer | ID | Sequence (5'->3') |
|---|---|---|
| Forward | SEQ ID NO: 5 | GCA GGA CAA GAA GAG TTT GGA |
| | SEQ ID NO: 6 | TCC ATG AAC TTG TCC GGG TT |
| Reverse | SEQ ID NO: 7 | TGA AGC AGC CTT AGT GTT TCC TT |

It was found that in the AHSB cohort, the frequency of the canonical allele in homozygosity (GG) at the SNP position was extremely low (4%) a frequency that is not at a Hardy-Weinberg equilibrium ( fig.2 A). This suggested that the scarcity of GG homozygotes within the tumoral samples is not due to a low frequency of the canonical G allele but to a selection against homozygosity in the tumoral AHSB cohort. In other words, according to the observed frequencies, either women GG homozygous at the rs8570 position have a very low tendency to develop BC or there are somatic mutations at that position in the tumors.

The inventors also sequenced a total of 125 samples of surrounding non-tumoral mammary tissue taken at the time of surgery, in addition to the corresponding tumor samples. After sequencing, the 3'-UTR of RRAS2, the inventors found again a very low frequency (3%) of GG homozygotes within the patient population, compared to the high frequency (90%) of GC heterozygotes (fig. 2B).

These data suggest that women born GG homozygotes have less probability of developing BC. However, the comparison of allele frequencies between tumoral and non-tumoral tissue within the AHSB cohort shows a much lower frequency of CC homozygotes in the non-tumoral tissue than in the tumoral ones (compare fig. 2A with 2B).

It was found that 209 out of a total of 378 (AHSB +FIVO cohorts) sequenced samples (a 55%) had C at the SNP position in both chromosomal gene alleles, whereas 156 (a 41%) were heterozygotes (GC) at the SNP position and only 13 (a 3%) was homozygote for the canonical allele (fig.2C).

It was found that 68 out of a total of 243 sequenced samples (a 28%) had C at the SNP position in both chromosomal gene alleles, whereas 165 (a 68%) were heterozygotes (GC) at the SNP position and only 10 (a 4%) was homozygote for the canonical nucleotide (GG, Figure 2A).

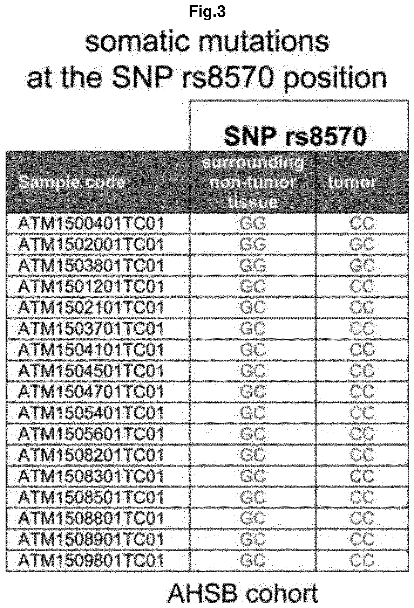

The distribution of GG and CC homozygotes and GC heterozygotes was not at equilibrium. A Hardy-Weinberg analysis of the frequency of the G and C alleles in the global population of breast tumor samples shows that the under-representation of GG homozygotes is not random (figure 2 A-C). A pairwise comparison of the sequences in the 107 patients with matched samples (surrounding non-tumoral samples) showed the presence of somatic mutations in the tumor samples resulting in an increased CC homozygosity in the tumoral samples (fig. 3).

The very low frequency of GG homozygotes within both the tumoral samples and the non-tumoral samples sequenced from breast cancer tumor patients contrasted with the high-frequency found in blood samples of 125 healthy volunteers of the Centro de Biologia Molecular (60 samples were GG, 48%, CBM cohort, figure 2D). The frequency of GG homozygotes was much higher in CBM cohort than that observed in the tumoral samples, and was at genetic Hardy-Weinberg equilibrium. These data suggest that people homozygote for G at the SNP rs8570 position are protected against developing breast cancer, at least of the types represented in the AHSB cohort.

A contingency test of homozygote distribution (GG vs CC) in the patient (AHSB+FIVO) versus healthy volunteer cohorts showed a very strong disequilibrium in the number of GG homozygotes in favor of CC homozygotes within the tumoral samples (CC to GG Odds Ratio=64.3; 95 CI=28.8-132.6).

### Example 2. Determination of the genotype at position of the SNP rs8570 from genomic DNA by PCR method.

For the rapid identification of GG or CC homozygotes and GC heterozygotes from small amounts of biological samples, quantitative PCR system based on the use of mismatching oligonucleotides that allow the quantification of genomic DNA bearing the G nucleotide and the C nucleotide at SNP 8570 in the 3'-UTR region of gene RRAS2 was set up. The mismatching primers are described in table 1. Both quantifications with mismatching primers were normalized to the amplification of exons 3-4 in the coding sequence wherein said amplification of exons 3-4 was carried out by using the primers of table 3.

**Table 3. Primers to measure RRAS2 expression amplification of exons 3-4.**

| Primer | ID | Sequence (5'->3') |
|---|---|---|
| Forward | SEQ ID NO: 8 | GCA GGA CAA GAA GAG TTT GGA |
| Reverse | SEQ ID NO: 9 | TCA TTG GGA ACT CAT CAC GA |

Both PCR reactions were performed using 30 cycles of 45 s at 95 °C, 45 s at 60 °C and 2 min at 72 °C with PfuTurbo DNA Polymerase.

As an example, the inventors showed a plot of normalized qPCRs for the G allele and for the C allele for genomic DNA extracted from capillary blood of healthy donors that had been previously sequenced and classified as of GG, GC or CC genotypes by Sanger sequencing. The results allow to perfectly group the samples from GC heterozygote donors in the diagonal of the plot and of GG and CC donors to each side of the diagonal (Figure 4).

### Example 3. Relationship between SNP rs8570 and RRAS2 overexpression and breast cancer.

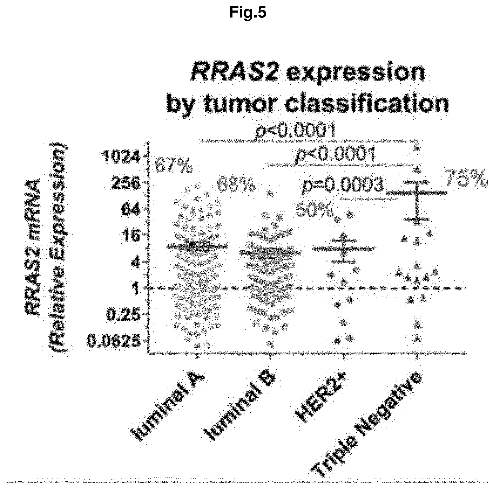

The analysis by RT-qPCR of mRNA expression in breast cancer samples from AHSB +FIVO cohorts showed that RRAS2 was overexpressed in the large majority of the tumors regardless their molecular classification into Luminal A, Luminal B, HER2+ or Triple Negative. However, the analysis of overexpression according to their molecular classification showed that RRAS2 was significantly more overexpressed in TNBC BCs than in the other three types (fig.5). Overexpression of RRAS2 was detected in 67% of Luminal A samples, 68% of Luminal B, 50% of Her2+ and 75% of the TNBC tumors (fig. 5).

The analysis of RRAS2 mRNA expression according to the genotype at the SNP rs8570 position showed a mean overexpression of RRAS2 of 35-fold, above mean expression in non-tumoral mammary gland tissue, in CC homozygotes versus 7-fold and 8-fold in GC heterozygotes and GG homozygotes, respectively (fig. 6).

The inventors established a statistically significant correlation between cancer samples in the AHSB+FIVO cohorts CC homozygotes for the SNP rs8570 position and the percentage of tumor cells expressing the proliferation marker Ki67, compared to GC heterozygotes. However, the number of samples with GG composition was too low as to draw any conclusion (fig. 7). Ki67 is a widely used markers of cells in proliferation and a higher percentage of Ki67+ cells is associated to more aggressive breast cancers. Therefore, the expression of the C allele in homozygosis (CC) at the SNP rs8570 position is associated with higher proliferation rate and therefore more aggressive disease. Another marker of poor prognosis analyzed for AHSB cohort was the loss of the tumor suppressor p53 (FIVO did not include such data). Tumor samples CC homozygotes were more highly represented within p53-negative tumors, compared to p53-positive ones (table 4).

**Table 4. Genotype at rs8570 and presence or absence of p53 in breast cancer samples**

| Genotype | p53 Negative samples | p53 Positive samples |
|---|---|---|
| GG | 5 (9%) | 3 (9%) |
| GC | 29 (51%) | 21 (60%) |
| CC | 23 (40%) | 11 (31%) |

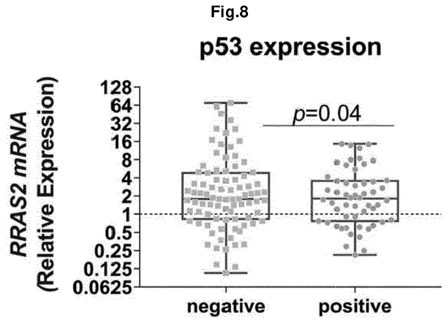

Interestingly, the analyses of RRAS2 mRNA expression within p53-negative and p53-positive tumors in the AHSB cohort showed that the former has significantly higher overexpression of RRAS2 than the latter (Fig. 8).

Although, the inventors could not gather information on patient survival within the AHSB and FIVO cohorts, metadata analysis allowed to establish a link between breast tumors with the highest overexpression of RRAS2 and short life-expectancy after diagnosis. The analysis of metadata in the METABRIC study showed that BC patients who survived less than 4 years after diagnosis had significantly more RRAS2 expression than those who survived up to 20 years after diagnosis. Finally, very long survivors (>23 years after diagnosis) were the group of patients with the lowest expression of RRAS2 in their tumors (Fig. 9). Altogether, these results associate expression of the C allele in homozygosis with higher RRAS2 expression in the tumors and with poorer prognosis.

Data were obtained from the Molecular Taxonomy of Breast Cancer International Consortium (METABRIC) cohort consists of 1980 patients with primary breast cancer. Copy number and gene expression from tumor banks-collected fresh frozen tissue was measured and deposited to the European Genome-Phenome Archive. The METABRIC database was acquired from the cBioPortal website (http://www.cbioportal.org/).

Statistical analyses were carried out using GraphPad Prism software (version 6.0). The number of biological replicates (n) and the statistical significance are indicated for each experiment in the figure legends. Significant differences between samples were assessed using the Student t test. P values <0.05 were considered statistically significant. Data obtained are given as the mean ± SD.

### Example 4. Allele at the SNP rs8570 position in RRAS2 mRNA and expression thereof.

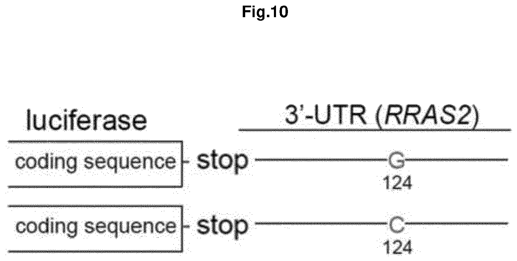

To interrogate if the G or C at the SNP rs8570 position determines gene expression levels, the inventors designed a reporter expression plasmid in which the coding sequence of the enzyme firefly luciferase is followed the stop codon (UAG) by the entire 3-UTR of RRAS2 with either G or C at the position of the SNP (124 nucleotides after the stop codon, figure 10).

For the plasmid generation RRAS2 3'-UTR sequences harvesting the G or the C allele in homozygosis at the rs8570 position were cloned by Gibson Assembly (New England Biolabs) into the firefly luciferase plasmid pGL3-Basic luciferase reporter. The cloning strategy removed the intrinsic polyadenylation signal from the pGL3-Basic vector, keeping only the polyA signal from RRAS2 3'-UTR. Clones of plasmids were replicated in DH5α competent E. coli cells and screened by DNA sequencing.

The two constructs were transfected into the human breast cancer cell line CAL-51 and luciferase activity was measured in cell lysates, 48 hours after transfection. The results showed that the construct with C at the SNP position expressed approximately 3.5-fold higher levels of luciferase activity than the construct with G (fig.11A).

For the Luciferase activity assays the cell lines were seeded in 24-well plates.48 hours after transfection in three biological replicates, cells were washed with phosphate-buffered saline (PBS, pH 7.4) and treated with Passive Lysis buffer from Promega Dual-luciferase reporter^{®} assay system. Luciferase activities were measured in a luminometer according to the manufacturer's instructions.

A quantitation of mRNA for the luciferase coding sequence by RT-qPCR showed that the construct bearing a C was expressed at concentrations 7-fold higher than the construct bearing a G (fig. 11B), thereby indicating that a C at the SNP position results in higher concentrations of mRNA. Luciferase expression was measured with primers of table 5 and normalized to 18S rRNA abundance using the 2^^(-ΔΔCt) method.

**Table 5. Primers to measure Luciferase and 18S rRNA expression**

| Primer | Gene | ID | Sequence (5'->3') |
|---|---|---|---|
| Forward | Luciferase | SEQ ID NO: 10 | TCA AAG AGG CGA ACT GTG |
| Reverse | Luciferase | SEQ ID NO: 11 | GGT GTT GGA GCA AGA TGG A |
| Forward | 18S rRNA | SEQ ID NO: 12 | ATC CAT TGG AGG GCA AGT C |
| Reverse | 18S rRNA | SEQ ID NO: 13 | GCT CCC AAG ATC CAA CTA CG |

## Claims

1. An *in vitro* use of the single nucleotide polymorphism (SNP) rs8570 of the mRNA of the gene *RRAS2* or the gDNA of the gene *RRAS2* for the prognosis of a patient suffering from breast cancer or for assessing the risk of developing breast cancer in a subject.

2. An *in vitro* method for the prognosis of a patient suffering from breast cancer, comprising the following steps:
i) detecting and genotyping the SNP rs8570 of the mRNA of the gene RRAS2 or of the gDNA of the gene RRAS2 in an isolated biological sample of said patient suffering from breast cancer;
ii) classifying the subject into a group of poor prognosis when at least one allele at SNP rs8570 of the RRAS2 gene is C.

3. An *in vitro* method according to claim 2, wherein the subject is classified into a group of poor prognosis in step ii) when the genotype at SNP rs8570 of the RRAS2 gene is CG or CC.

4. An *in vitro* method according to claim 2 or 3, wherein the genotype CG at SNP rs8570 of the gene RRAS2 indicates a poor prognosis with respect to the genotype GG at SNP rs8570 of the gene RRAS2; and the genotype CC at SNP rs8570 of the gene RRAS2 indicates poorer prognosis respect to the genotype CG.

5. The method according to any one of claims 2 to 4 wherein the biological sample is a tumoral sample, epidermal tissue, mucosal sample, whole blood, blood cells, serum, plasma, saliva, sputum, urine or breast exudate.

6. An *in vitro* method for assessing the risk of developing breast cancer in a subject, comprising the following steps:
i) detecting and genotyping the SNP rs8570 of the mRNA of the gene RRAS2 or of the gDNA of the gene RRAS2 in an isolated biological sample of said subject;
ii) classifying the subject into a group of increased risk of developing breast cancer when at least one allele at SNP rs8570 of the RRAS2 gene is C.

7. An *in vitro* method according to claim 6, wherein the subject is classified into a group of increased risk of developing breast cancer in step ii) when the genotype at SNP rs8570 of the RRAS2 gene is CG or CC.

8. An *in vitro* method according to any of claims 6 or 7, wherein the step ii) further classifies the subject into a group of decreased risk of developing breast cancer when the genotype at SNP rs8570 of the RRAS2 gene is GG.

9. An *in vitro* method according to any one of claims 6 to 8, wherein the genotype CG at SNP rs8570 of the gene RRAS2 indicates an increased risk of developing breast cancer with respect to the genotype GG at SNP rs8570 of the gene RRAS2; and the genotype CC at SNP rs8570 of the gene RRAS2 indicates an increased risk of developing breast cancer with respect to the genotype CG.

10. The method according to any of one of claims 6 to 9 wherein the biological sample is an epidermal tissue, mucosal sample, whole blood, blood cells, serum, plasma, saliva, sputum, urine or breast exudate.

11. The *in vitro* use according to claim 1 or the *in vitro* method according to any one of claims 2 to 10 wherein the breast cancer is selected from the group consisting of: Luminal A breast cancer, Luminal B breast cancer, Her2+ breast cancer or triple Negative Breast Cancer (TNBC).

12. The *in vitro* method according to any one of claims 2 to 11 wherein the detection and genotyping of the SNP is performed out by means of quantitative polymerase chain reaction (qPCR), Real-Time PCR (RT-qPCR), Retro-Transcriptase PCR (RT-PCR), long-range PCR, Restriction Fragment Length Polymorphism-PCR (PCR-RFLP), Loop-mediated isothermal amplification (LAMP), allele specific-LAMP amplification (AS-LAMP), reverse transcriptase-LAMP (RT-LAMP), DNA microarray, RNA microarray, oligonucleotide hybridization technique, Locked nucleic acid hybridization (LAN) and/or sequencing method, preferably wherein the sequencing method is Sanger method.

13. A kit for assessing the risk of developing breast cancer in a subject or for the prognosis of a patient suffering from breast cancer, wherein the kit comprises oligonucleotides and probes specific for the detection and genotyping of the SNP rs8570 of the mRNA of the gene RRAS2 or of the gDNA of the gene RRAS2, preferably wherein the kit comprises the oligonucleotides comprising, more preferably consisting of, the SEQ ID NO: 2, SEQ ID NO: 3 and SEQ ID NO: 4.

14. An *in vitro* use of the kit according to claim 13 for predicting the risk of developing breast cancer in a subject or for the prognosis of a patient suffering from breast cancer.
